# EUROPEAN PATENT SPECIFICATION

(11) **EP 2 709 644 B1**
(45) Date of publication and mention of the grant of the patent: **13.03.2019**
(21) Application number: 12786404.9
(22) Date of filing: 26.01.2012
(51) Int. Cl.: A61K 38/16, A61P 31/04, A61K 38/13, A61K 38/14, A61K 45/06, A61P 11/00, A61K 47/44, A61K 31/496, A61K 31/7048

(54) **COMPOSITIONS FOR USE FOR TREATING A DISORDER ASSOCIATED WITH SEQUESTERED BACTERIA**
ZUSAMMENSETZUNGEN ZUR VERWENDUNG ZUR BEHANDLUNG EINER ERKRANKUNG IM ZUSAMMENHANG MIT SEQUESTRIERTEN BAKTERIEN
COMPOSITIONS POUR L'UTILISATION DANS LE TRAITEMENT D'UNE AFFECTION ASSOCIÉE À DES BACTÉRIES SÉQUESTRÉES

(30) Priority: 16.05.2011 US 201161486566 P
(43) Date of publication of application: 26.03.2014
(73) Proprietor: Beech Tree Labs Inc., Delanson, NY 12053 (US)
(72) Inventor: MCMICHAEL, John, Delanson, NY 12053 (US)
(74) Representative: Patentanwälte Olbricht Buchhold Keulertz
(86) International application number: PCT/US2012/022764
(87) International publication number: WO 2012/158216

(56) References cited:
- US-A1- 2007 219 119
- US-A1- 2009 162 392
- US-A1- 2009 291 964
- US-A1- 2010 144 602
- DAVID W HOROHOV ET AL: "The use of streptolysin O (SLO) as an adjunct therapy forpneumonia in foals", VETERINARY MICROBIOLOGY, ELSEVIER BV, NL, vol. 154, no. 1, 30 June 2011 (2011-06-30) , pages 156-162, XP028104188, ISSN: 0378-1135, DOI: 10.1016/J.VETMIC.2011.06.037 [retrieved on 2011-07-07]

## Description

### BACKGROUND OF THE INVENTION

An abscess is a cavity containing pus and surrounded by inflamed tissue, formed as a result of a localized infection. In the lung, an abscess may occur in the setting of a pneumonia. Abscesses can be caused by minor breaks and punctures of the skin, obstruction of sweat glands and oil (sebaceous) glands, and inflammation of hair follicles. They contain dead cells, bacteria, and other debris, which causes inflammation and pain. Common bacteria, such as staphylococci, are the most common cause, although the bacillus responsible for tuberculosis is an important abscess-forming type. Antibiotics are usually prescribed to treat a bacterial infection, however, the lining of the abscess cavity tends to reduce the amount of drug that can penetrate the source of infection from the bloodstream.

Infection with *Rhodocaccus equi* leads to subacute or chronic abscessating bronchopneumonia in young foals. Standard antimicrobial therapy consists of a long term course of antibiotic therapy using rifampin and macrolide antibiotic (erythromycin or others). Although this combined therapy has dramatically improved the survival rate of foals infected with *R. equi,* this treatment regimen is not without problems. Erythromycin has variable absorption in foals when given orally and requires multiple daily dosing, and most importantly, its administration has a high incidence of potentially fatal side effects (Jacks, S. S., Giguere, S., Nguyen, A, 2003, In vitro susceptibilities of Rhodococcus equi and other common equine pathogens to azithromycin, clarithromyein, and 20 other antimicrobials. Antimicrob Agents Chemother 4 1742-1745). While azithromycin and clarithromycin have been proposed as alternatives as they are more chemically stable, have a greater bioavailability, and achieve higher concentrations in phagocytic cells and tissues than erythromycin (Jacks et al., 2003, supra), the ability of antibiotics to penetrate granulomas containing the bacteria and infected macrophages is of concern. While residual pulmonary scarring is not typically observed post-infection (Martens, R.J., Martens, 3,G., Fiske, RA., Hietala, S.K., 1989, Rhodococcus equi foal pneumonia: protective effects of immune plasma in experimentally infected foals. Equine Vet J 21, 249-255), *R. equi* infection in foals is associated with a decreased chance of racing as an adult (Ainsworth et al., Associations between physical examination, laboratory, and radiographic findings and outcome and subsequent racing performance of foals with Rhodococcus equi infection: 115 cases (1984-1992), J Am Vet Med Assoc 213, 510-515, 1998). These results suggest that even in those foals which respond to standard therapy, some deficit remained. Enhancing the response to therapy could reduce this detrimental effect of infection. Thus, there remains a need in the art for an improved therapy for abscessating infections.

Streptolysin O is one of a group of filterable hemolysins derived from Group A beta-hemolytic streptococci. Specifically, streptolysin O is a 60-kD peptide, which is hemolytic in its reduced state, but is inactivated upon oxidation (Johnson et al., Infect. Immun., 27:97-101, 1980; Alouf et al., Pharmacol. Ther., 3:661-717, 1984; Bhakdi et al., Infect. Immun., 47:52-60, 1985). Group A streptococci produce streptolysin O. Streptolysin O is used in the art generally as an analytical reagent for permeabilizing cells (e.g. Razin et al., Proc. Nat'l. Acad. Sci. (USA) 91:7722-7726 (1994).

. U.S. Patent No. 5,576,289 discloses the use of streptolysin O in methods for treating disease states characterized by motor deficit including multiple sclerosis and autism. U.S. Patent No. 5,736,508 discloses the use of streptolysin O in methods for treating scarring. Neither the '289 patent nor the '508 patent disclose or suggest the use of streptolysin O for enhancing the anti-bacterial effects of an antibiotic against sequestered bacteria. U.S. Patent Application US 2012/144602 relates to methods for treatment of various conditions by the administration of streptolysin O. The publication of DAVID W HOROHOV ET AL: "The use of streptolysin O (SLO) as an adjunct therapy for pneumonia in foals", VETINARY MICROBIOLOGY, ELSEVIER BV, NL, vol. 154, no. 1, 30 June 2011 (2011-06-30), pages 156-162, XP028104188, ISSN: 0378-1135, DOI: 10.1016/J.VETMIC.2011.06.037 describes the use of streptolysin O in treating pneumonia caused by *Rhodococcus equi.* U.S. Patent application US 2009/162392 relates to the use of forms of streptolysin O in vaccine compositions to induce protection against S. pyogenes. U.S. Patent application US 2009/291964 describes compounds that potentiate the activity of antibacterials and compositions useful in treating bacterial infection in mammals. U.S. Patent application US 2007/219119 relates to methods for administering streptolysin O for treatment of various conditions and for protecting nerve cells from the effects of neurotoxic agents by the administration of streptolysin O.

### SUMMARY OF THE INVENTION

The present application relates to the discovery that treatment of infections associated with sequestered bacteria with a combination therapy comprising streptolysin O and antibiotic(s) is more effective than treatment with antibiotic(s) alone. Described herein is a method of treating a disorder associated with sequestered bacteria in a mammalian subject comprising the step of administering to the subject a combination therapy comprising streptolysin O (SLO) and an antibiotic wherein the combination therapy is administered in amounts effective to treat the disorder. Also contemplated is combination therapy comprising streptolysin O and an antibiotic for use in the treatment of a disorder associated with sequestered bacteria in a mammalian subject, wherein the combination therapy is administered in an amount effective to treat the disorder. **All embodiments encompassing a method of therapy or treating a disorder are excluded from the claims.**

In some embodiments, the streptolysin O and antibiotic are administered concurrently. Sequential administration of the combination therapy is also contemplated.

In some embodiments, the sequestered bacteria is selected from the group consisting of *H. influenza, N. meningitidis, S. pneumoniae, M. tuberculosis, M. pneumonia, S. aureus* and *R. equi.* In some embodiments, the sequestered bacteria is a member of the Staphylococcus species selected from the group consisting of *S. aureus, S. pyogenes, S. intermedius, S. epidermidis, S. haemolyticus, S. saprophyticus, S. sciuri, S. simulans* and *S*. *warneri.*

In some embodiments, the disorder is selected from the group consisting of infection associated with mastitis, a pyoderma (including, but not limited to, ecthyma, feruncles and carbuncles), tuberculosis (e.g., of the bone or lung) and pneumonia. In some embodiments, the disorder is an infection associated with mastitis and the subject to be treated is selected from the group consisting of a cow and a goat. In other embodiments, the disorder is a pyoderma and the subject to be treated is selected from the group consisting of a dog, a cat, a cow, and a goat.

The antibiotic for use in the combination therapy may comprise any one or more antibiotics useful in treating a disorder associated with sequestered bacteria. In some embodiments, the antibiotic comprises a member selected from the group consisting of rifampin, ciproflaxin, enoxacin, gatifloxacin, levofloxacin, lomefloxacin, moxifloxacin, nalidixic acid, norflonacin, oflonacin, capreomycin, cycloserine, ethambutol, thionamide, isoniazid, pyrazinamide, streptomycin, rclarithromycin, ifapentine, chloramphenicol, metroidazole, thiamphenicol, ertapenem, doripenem, cilastatin, doxycycline, erythromycin, nafcillin, oxacillin, vancomycin, penicillin and combinations thereof. In one embodiment, the antibiotic comprises a combination of rifampin and clarithromycin.

The streptolysin O is, in some embodiments, formulated in a number of pharmaceutically-acceptable carriers or excipients including, but not limited to, water, saline, albumin, dextrose or any other pharmaceutically acceptable excipient known in the art. The precise dose will vary among patients and may readily be determined by those of ordinary skill in the art. In some embodiments, the streptolysin O is administered in a dosage amount ranging from 0.0032 to 50 units (2 units/0.05 ml) per day and is preferably formulated in a liquid vehicle. In some embodiments, the streptolysin O is provided at a concentration of approximately 4 units as a single drop. A single drop of streptolysin O is within the range of 0.05 to 10 units. In some embodiments, a drop of streptolysin O is in the amount of 2 units as a single drop. In other embodiments, the streptolysin O is more administered in an amount ranging from 0.01 to 10 units per day. In still other embodiments, the streptolysin O is administered in an amount ranging from about 0.1 to 8 units per day. In other embodiments, the administered dose of streptolysin O is from about 1 unit to about 5 units. In yet other embodiments, the administered dose of streptolysin O is about 2 units. A preferred route of administration is subcutaneous injection, but other routes, such as bucal, oral drench, sublingual, intradermal, intramuscular, intrathecal, intravenous, inhalation or topical, are also contemplated. For non-human animals such as horses, a preferred mode of administration is by subcutaneous administration at a dosage of 2 units per dose (0.2 cc).

In a composition for use in treating a disorder associated with sequestered bacteria in a mammalian subject, the disorder is an infection selected from the group consisting of mastitis, a pyoderma, tuberculosis and pneumonia, the invention lies in that the composition comprises streptolysin O (SLO) and an antibiotic in amounts effective to treat the disorder in a combination therapy. According to one aspect of the invention the composition for use comprises SLO in an amount from ranging from 0.0008 to 50 units with amounts ranging from 0.025 to 10 units being preferred and of about 2 units being particularly preferred.

Preferred compositions for use include those wherein the antibiotic comprises an antibiotic selected from the group consisting of rifampin, ciproflaxin, enoxacin, gatifloxacin, levofloxacin, lomefloxacin, moxifloxacin, nalidixic acid, norflonacin, oflonacin, capreomycin, cycloserine, ethambutol, thionamide, isoniazid, pyrazinamide, streptomycin, rclarithromycin, ifapentine, chloramphenicol, metroidazole, thiamphenicol, ertapenem, doripenem, cilastatin, doxycycline, erythromycin, nafcillin, oxacillin, vancomycin, penicillin and combinations thereof with combinations of rifampin and clarithromycin being particularly preferred. According to one aspect of the invention a composition for use comprising from 0.5 to 4 units SLO, from 200 to 500 mg rifampin and from 250 to 600 mg clarithromycin is preferred for administration one or several times per day for treatment of sequestered bacteria such as tuberculosis (*Mycobacterium tuberculosis*).

Compositions for use according to the invention can further comprise a a pharmaceutically acceptable excipient such as those wherein the pharmaceutically acceptable excipient is selected from the group consisting of proteins, polysaccharides, polylactic acids, polyglycolic acids, polymeric amino acids, amino acid copolymers, and inactive virus particles, antioxidants, chelating agents carbohydrates, dextrin hydroxyalkylcellulose, hydroxyalkylmethylcellulose, stearic acid, oils, saline, glycerol, ethanol, wetting agents, emulsifying agents, pH buffering substances and liposomes. According to one aspect of the invention the pharmaceutically acceptable excipient is a sterile fixed oil.

Additional aspects, features and variations of the invention will be apparent from the entirety of this application, including the detailed description, and all such features are intended as aspects of the invention. It should be understood, however, that the detailed description and the specific examples are given by way of illustration, and that the many various changes and modifications that will be apparent to those familiar with the field of the invention are also part of the invention.

Aspects of the invention described with the term "comprising" should be understood to include the elements explicitly listed, and optionally, additional elements. Aspects of the invention described with "a" or "an" should be understood to include "one or more" unless the context clearly requires a narrower meaning.

### DETAILED DESCRIPTION OF THE INVENTION

The present application relates to the discovery that treatment of infections associated with sequestered bacteria with a combination therapy comprising streptolysin O and antibiotic therapy is more effective than treatment with antibiotic therapy alone. Without wishing to be bound by any theory, it is contemplated that streptolysin O enhances the antibiotic therapy by breaking down the abscesses associated with many pathogenic bacteria allowing the antibiotics to have access to the bacteria within the abscess.

In one aspect, described herein is a method of treating a disorder associated with sequestered bacteria in a mammalian subject comprising the step of administering to the subject a combination therapy comprising streptolysin O and antibiotic therapy in an amount effective to treat the disorder. In some embodiments, the disorder to be treated is a lower respiratory infection. In one embodiment, the disorder is selected from the group consisting of tuberculosis, pneumococcal pneumonia, bronchitis, and bacterial pneumonia due to agents other than *S. pneumonia* (e.g., *K. pneumonia, M. pneumonia, L. pneumonia, C. pneumonia).*

The term "effective amount" as used herein, refers to an amount of the combination therapy (i.e., streptolysin O and antibiotic therapy) sufficient to treat, ameliorate, or prevent the identified disease or condition, or to exhibit a detectable therapeutic, prophylactic, or inhibitory effect. The effect can be detected by, for example, an improvement in clinical condition or a reduction in symptoms. The precise effective amount for a subject will depend upon the subject's body weight, size, and health; the nature and extent of the condition; and the antibiotic therapy selected for administration. Therapeutically effective amounts for a given situation can be determined by routine experimentation that is within the skill and judgment of the clinician.

The term "streptolysin O" as used with respect to the methods described herein means streptolysin O which has been modified by oxidation to eliminate cytotoxic effects while retaining important cholesterol binding characteristics on the cell membrane. Streptolysin O is readily oxidized in solution and is commercially available (Sigma Product Catalog).

The dose of streptolysin O administered to the subject can be determined by the physician, taking into account, age, sex, weight, etc. of the subject. In some embodiments, the administered dose of streptolysin O is at least 0.01, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3.0, 4.0, 5.0, 6.0, 7.0, 8.0, 9.0, 10.0 or more International units. In one embodiment, the administered dose of streptolysin O is 2 units. In some embodiments, streptolysin O is administered 1, 2, 3, 4, 5, 6, 7, 8, 9, 10 or more times daily for a period of 1, 2, 3, 4, 5, 6 or more weeks. Additional therapy may be administered on a period basis, for example, daily, weekly or monthly.

The use of any standard of care antibiotic therapy known in the art is contemplated. "Standard of care antibiotic therapy" is therapy with one or more antibiotics that is generally accepted by medical clinicians for a certain type of patient diagnosed with a particular type of bacterial infection. Exemplary antibiotics include, but are not limited to, penicillins, cephalosporins, carbacephems, cephamycins, carbapenems, monobactams, aminoglycosides, glycopeptides, quinolones, tetracyclines, macrolides, and fluoroquinolones. Examples of antibiotic agents include, but are not limited to, Penicillin G (CAS Registry No.: 61-33-6); Methicillin (CAS Registry No.: 61-32-5); Nafcillin (CAS Registry No.: 147-52-4); Oxacillin (CAS Registry No.: 66-79-5); Cloxacillin (CAS Registry No.: 61-72-3); Dicloxacillin (CAS Registry No.: 3116-76-5); Ampicillin (CAS Registry No.: 69-53-4); Amoxicillin (CAS Registry No.: 26787-78-0); Ticarcillin (CAS Registry No.: 34787-01-4); Carbenicillin (CAS Registry No.: 4697-36-3); Mezlocillin (CAS Registry No.: 51481-65-3); Azlocillin (CAS Registry No.: 37091-66-0); Piperacillin (CAS Registry No.: 61477-96-1); Imipenem (CAS Registry No.: 74431-23-5); Aztreonam (CAS Registry No.: 78110-38-0); Cephalothin (CAS Registry No.: 153-61-7); Cefazolin (CAS Registry No.: 25953-19-9); Cefaclor (CAS Registry No.: 70356-03-5); Cefamandole formate sodium (CAS Registry No.: 42540-40-9); Cefoxitin (CAS Registry No.: 35607-66-0); Cefuroxime (CAS Registry No.: 55268-75-2); Cefonicid (CAS Registry No.: 61270-58-4); Cefmetazole (CAS Registry No.: 56796-20-4); Cefotetan (CAS Registry No.: 69712-56-7); Cefprozil (CAS Registry No.: 92665-29-7); Lincomycin (CAS Registry No.: 154-21-2); Linezolid (CAS Registry No.: 165800-03-3); Loracarbef (CAS Registry No.: 121961-22-6); Cefetamet (CAS Registry No.: 65052-63-3); Cefoperazone (CAS Registry No.: 62893-19-0); Cefotaxime (CAS Registry No.: 63527-52-6); Ceftizoxime (CAS Registry No.: 68401-81-0); Ceftriaxone (CAS Registry No.: 73384-59-5); Ceftazidime (CAS Registry No.: 72558-82-8); Cefepime (CAS Registry No.: 88040-23-7); Cefixime (CAS Registry No.: 79350-37-1); Cefpodoxime (CAS Registry No.: 80210-62-4); Cefsulodin (CAS Registry No.: 62587-73-9); Fleroxacin (CAS Registry No.: 79660-72-3); Nalidixic acid (CAS Registry No.: 389-08-2); Norfloxacin (CAS Registry No.: 70458-96-7); Ciprofloxacin (CAS Registry No.: 85721-33-1); Ofloxacin (CAS Registry. No.: 82419-36-1); Enoxacin (CAS Registry No.: 74011-58-8); Lomefloxacin (CAS Registry No.: 98079-51-7); Cinoxacin (CAS Registry No.: 28657-80-9); Doxycycline (CAS Registry No.: 564-25-0); Minocycline (CAS Registry No.: 10118-90-8); Tetracycline (CAS Registry No.: 60-54-8); Amikacin (CAS Registry No.: 37517-28-5); Gentamicin (CAS Registry No.: 1403-66-3); Kanamycin (CAS Registry No.: 8063-07-8); Netilmicin (CAS Registry No.: 56391-56-1); Tobramycin (CAS Registry No.: 32986-56-4); Streptomycin (CAS Registry No.: 57-92-1); Azithromycin (CAS Registry No.: 83905-01-5); Clarithromycin (CAS Registry No.: 81103-11-9); Erythromycin (CAS Registry No.: 114-07-8); Erythromycin estolate (CAS Registry No.: 3521-62-8); Erythromycin ethyl succinate (CAS Registry No.: 41342-53-4); Erythromycin glucoheptonate (CAS Registry No.: 23067-13-2); Erythromycin lactobionate (CAS Registry No.: 3847-29-8); Erythromycin stearate (CAS Registry No.: 643-22-1); Vancomycin (CAS Registry No.: 1404-90-6); Teicoplanin (CAS Registry No.: 61036-64-4); Chloramphenicol (CAS Registry No.: 56-75-7); Clindamycin (CAS Registry No.: 18323-44-9); Trimethoprim (CAS Registry No.: 738-70-5); Sulfamethoxazole (CAS Registry No.: 723-46-6); Nitrofurantoin (CAS Registry No.: 67-20-9); Rifampin (CAS Registry No.: 13292-46-1); Mupirocin (CAS Registry No.: 12650-69-0); Metronidazole (CAS Registry No.: 443-48-1); Cephalexin (CAS Registry No.: 15686-71-2); Roxithromycin (CAS Registry No.: 80214-83-1); Co-amoxiclavuanate; combinations of Piperacillin and Tazobactam; and their various salts, acids, bases, and other derivatives.

In some embodiments, the antibiotic comprises one or more antibiotics selected from the group consisting of rifampin, ciproflaxin, enoxacin, gatifloxacin, levofloxacin, lomefloxacin, moxifloxacin, nalidixic acid, norflonacin, oflonacin, capreomycin, cycloserine, ethambutol, thionamide, isoniazid, pyrazinamide, streptomycin, rclarithromycin, ifapentine, chloramphenicol, metroidazole, thiamphenicol, ertapenem, doripenem, cilastatin, doxycycline, erythromycin, nafcillin, oxacillin, vancomycin, penicillin and combinations thereof. In one embodiment, the antibiotic therapy comprises rifampin and clarithromycin.

The amount of antibiotic administered to the subject can be readily determined by the physician, taking into account, age, sex, weight, etc. of the subject. Typically, a suitable dosage is about 0.5 to 40 mg/kg body weight, or about 1 to 30 mg/kg, or about 1 to 25 mg/kg, or about 1 to 20 mg/kg, or about 1 to 20 mg/kg, or about 1 to 15 mg/kg, or about to 10 mg/kg or about 1 to 9 mg/kg, or about 1 to 8 mg/kg, or about 1 to 7 mg/kg, or about 1 to 6 mg/kg, or about 1 to 5 mg/kg body weight.

In some embodiments, the streptolysin O and antibiotic(s) are administered to the subject concurrently. In other embodiments, the streptolysin O and antibiotic(s) are administered sequentially. For example, administration of streptolysin O may precede or follow the antibiotic(s) by intervals ranging from minutes to weeks. In embodiments where the antibiotic(s) and streptolysin O are administered separately, one would generally ensure that a significant period of time did not expire between the times of each delivery, such that the antibiotic(s) and the streptolysin O would still be able to exert an advantageously combined effect. In such instances, it is contemplated that one would administer both modalities within about 12-24 hours of each other. In some embodiments, the modalities would be administered within 6-12 hours of each other. In other embodiments, the modalities are administered within 1-6 hours of each other. Repeated treatments with one or both agents are specifically contemplated. In some embodiments, streptolysin and the antibiotic(s) is delivered directly to the subject or in compositions along with suitable carriers or excipients, as is well known in the art.

The subjects treated in the methods disclosed herein in its many embodiments are desirably human subjects, although it is to be understood that the principles of the presently disclosed subject matter indicate that the presently disclosed subject matter is effective with respect to invertebrate and to all vertebrate species, including mammals, which are intended to be included in the term "subject."

The streptolysin O and/or antibiotic(s) described herein may be formulated in pharmaceutical compositions with a pharmaceutically acceptable excipient, carrier, or diluent.

Administration may take the form of single dose administration, or the compound of the embodiments can be administered over a period of time, either in divided doses or in a continuous-release formulation or administration method (*e*.*g*., a pump). However the compounds of the embodiments are administered to the subject, the amounts of compound administered and the route of administration chosen should be selected to permit efficacious treatment of the disease condition.

In some embodiments, the pharmaceutical compositions are formulated with pharmaceutically acceptable excipients such as carriers, solvents, stabilizers, adjuvants, diluents, etc., depending upon the particular mode of administration and dosage form. The pharmaceutical compositions should generally be formulated to achieve a physiologically compatible pH, and may range from a pH of about 3 to a pH of about 11, preferably about pH 3 to about pH 7, depending on the formulation and route of administration. In alternative embodiments, it may be preferred that the pH is adjusted to a range from about pH 5.0 to about pH 8. More particularly, the pharmaceutical compositions comprises in various aspects a therapeutically effective amount of at least one composition as described herein, together with one or more pharmaceutically acceptable excipients.

The term "pharmaceutically acceptable excipient" refers to an excipient for administration of a pharmaceutical agent, such as the compounds described herein. The term refers to any pharmaceutical excipient that may be administered without undue toxicity.

Pharmaceutically acceptable excipients are determined in part by the particular composition being administered, as well as by the particular method used to administer the composition. Accordingly, there exists a wide variety of suitable formulations of pharmaceutical compositions (see, *e*.*g*., Remington's Pharmaceutical Sciences).

Suitable excipients may be carrier molecules that include large, slowly metabolized macromolecules such as proteins, polysaccharides, polylactic acids, polyglycolic acids, polymeric amino acids, amino acid copolymers, and inactive virus particles. Other exemplary excipients include antioxidants (*e*.*g*., ascorbic acid), chelating agents (*e*.*g*., EDTA), carbohydrates (*e*.*g*., dextrin, hydroxyalkylcellulose, and/or hydroxyalkylmethylcellulose), stearic acid, liquids (*e*.*g*., oils, water, saline, glycerol and/or ethanol) wetting or emulsifying agents, pH buffering substances, and the like. Liposomes are also included within the definition of pharmaceutically acceptable excipients.

Additionally, the pharmaceutical compositions may be in the form of a sterile injectable preparation, such as a sterile injectable aqueous emulsion or oleaginous suspension. This emulsion or suspension may be formulated by a person of ordinary skill in the art using suitable dispersing or wetting agents and suspending agents. The sterile injectable preparation may also be a sterile injectable solution or suspension in a non-toxic parenterally acceptable diluent or solvent, such as a solution in 1,2-propane-diol.

Among the acceptable vehicles and solvents that may be employed are water, Ringer's solution, and isotonic sodium chloride solution. In addition, sterile fixed oils may be employed as a solvent or suspending medium. For this purpose any bland fixed oil may be employed including synthetic mono- or diglycerides. In addition, fatty acids (*e*.*g*., oleic acid) may likewise be used in the preparation of injectables.

### EXAMPLE

The following Example demonstrates that foals challenged with *R. equi* that were treated with a combination therapy comprising a standard antibiotic regimen (i.e., rifampin and clarithromycin) and SLO exhibited lower bacterial counts in the lung tissue examined at necropsy compared to the foals receiving either antibiotic therapy alone or SLO therapy alone. This result suggests that the SLO in the combination therapy enhanced the efficacy of the antibiotic treatment by increasing penetration into the congested lung or individual granulomas.

### Materials and Methods

*Animals:* 24 foals (average age 4 +/-3 days old) were used in this challenge model and two additional foals served as uninfected controls. The foals were raised on a Veterinary Science farm and kept with their mares throughout the study. Foals were evaluated shortly after birth for inclusion in the study using the following criteria; normality in physical examination, absence of lung sounds on auscultation, normal temperature, and unremarkable ultrasound examination of the lungs. None of the foals received anti-rhodococcal plasma or other treatments prior to enrollment in the study. The foals were moved with their dams to individual box stalls in a BSL2 isolation facility. Baseline values for heart rate, respiratory rate, temperature, fibrinogen concentration, and leukocyte count were obtained.

All foals were healthy at birth and obtained sufficient colostrums from the mare. In particular, 21 of the foals had plasma Ig concentrations; >800 mg/dl with the remaining 3 foals between 400 and 800 mg/dl. Based on these results, no Ig supplementation was deemed necessary. The average initial weight of the foals was 57.9 ± 8.5 kgs and there was no difference between groups. For all foals, the average percentage of peripheral blood mononuclear cells (PBMCs) producing IFN-γ was 2.72 ± 1.0 % and following group assignments, there was no difference between the groups.

*Challenge with R. equi:* Foals were challenged with an intrabronchial instillation of *R. equi* at an initial dose concentration of 2.2x10⁸ bacteria per foal and then reduced to ∼1.5x10⁵ bacteria per foal. The isolate was confirmed to contain the virulence-associate plasmid. Briefly, bacterial cultures were grown on agar plates overnight prior to inoculation, as previously described (Jacks, S., Giguere, S., 2010, Veterinary Immunology and Immunopathology 282-286).

After the foals were sedated, a flexible fiberoptic endoscope was used to deliver the bacteria suspended in 35 ml of sterile PBS into both main bronchi. The first six foals received 3.1±3.0 x 10⁸ bacteria and the remaining foals received 1.4 ±.01 x 10⁷ bacteria. The foals were returned to their stalls and dams and monitored daily for changes in rectal temperature, respiration, and pulse as determined by physical exam and auscultation of the lungs. Ultrasound scanning was performed twice weekly beginning one week after the inoculation and continuing throughout the time the foal remained on the study. Blood was obtained via jugular venipuncture on the day of each ultrasound scan for fibrinogen analysis. Clinical signs recorded included lung auscultations, overall attitude, appetite, resting respiratory rate and pulse. Foals with suspect lesions (>2 cm) identified by ultrasound or with any clinical abnormalities consistent with rhodococcal pneumonia were assigned to one of the treatment groups (see below). Daily observation of attitude, appetite, lung auscultation were scored (0-4) and combined to generate an overall daily score for each foal. Twice daily temperatures were collected over the sampling period. If the temperature was normal (<102.5°F) the temperature score for that day was scored "0", if >102.5, but less than 103.5, it was scored a "3." The average temperature score post treatment was then calculated by summing the daily temperature scores after treatment began.

*Treatment:* The three treatment groups were as follows: Antibiotic therapy combined with SLO therapy (Group A, n=8), SLO therapy (Group B, n=4) and standard antibiotic therapy alone (Group C, n=7), and non-infected, non-treated controls (n=2). The antibiotic treatment regimen consisted of clarithromycin (7.5 mg/kg) and rifampin (5 mg/kg) given per os twice daily. The administration of SLO in Groups A and B involved a 0.2 mL (2 IU) administration by subcutaneous injection twice a day. All treatments in Groups A-C were continued for 21 days unless symptoms progressed to the point where the foal needed to be euthanized.

The foals were observed daily throughout the treatment period. Foals were treated symptomatically for fever and other clinical signs. In the later stages of the infection, those foals exhibiting flared nostrils and extreme difficulty in breathing were euthanized early. After it was determined that those assigned to Group B (SLO alone) deteriorated rapidly and required early euthanasia, all subsequent foals were assigned to Groups A (SLO +antibiotic therapy) or C (antibiotic therapy alone), randomly. All surviving foals were euthanized 21 days after the start of treatment.

The investigator and the veterinarian treating the foals were blinded to the treatment received by each foal. The data were analyzed using the group designations prior to decoding. Differences between treatment groups were determined using two way analysis of variance (Systat Software, Inc., San Jose, CA). Statistical significance was determined at p<0.05. Since no treatment effect was observed in Group B (SLO alone), the results from these foals were combined with those of the non-treated group.

### Results

*Clinical findings*: Plasma fibrinogen was determined on days -2, 5, 12 and 19 post challenge. There was significant rise in fibrinogen levels post-infection peaking on day 12 in the high dose challenge group and on day 19 for the low dose challenge group. There was also a significant effect of challenge dose on the number of bacteria in the lungs at necropsy. Those foals receiving the higher challenge dose also exhibited clinical signs earlier than the lower challenge dose foals. However, the fact that disease developed sooner and that there were more bacteria in the lung did not equate with more severe clinical signs in the foals nor higher pneumonia scores at necropsy. Indeed for all other measures (except CFU/g lung tissue), dose of bacteria was not significant and therefore removed from the model as a variable.

Daily observation of attitude, appetite, lung auscultation and temperature were scored (0-4) and combined to generate an overall daily score for each foal. There was no effect of bacterial dose on clinical scores. There was a significant treatment effect (p=0.038) on the average clinical response, those foals in treatment Group A (antibiotic therapy + SLO) had lower clinical scores that the other treatment groups. Treatment had no significant effect (p=0.108) in regards to rectal temperature. There was no effect of bacterial dose on the febrile response. While the foals in Group A(antibiotic therapy + SLO) had fewer lung sounds at asculation, this was not significant.

*Necropsy findings:* Seven sections of lung were histophathologically evaluated and graded for each foal on the distribution, degree of pyogranulomatous inflammation, and alveolar necrosis. A pneumonia score was generated individually for each of the 7 sections and for the whole animal. Lung tissue samples were obtained for bacteriological culturing and RNA analyses. Peripheral blood samples were collected for assessment of *R. equi*-specific IFN-γ production. None of the foals' peripheral blood mononuclear cells (PBMCs) produced significant amounts of IFN-γ after *in vitro* stimulation with *R equi* prior to infection and there was no difference between groups in their response post-infection.

At necropsy, the weight gain (weight at necropsy minus weight at birth) in the Group A (SLO + antibiotic therapy) foals was higher than those of the other groups, however this failed to achieve statistical significance (p=0.057). Nor was there an effect of bacterial dose on weight gain. All foals exhibited multiple microgranulomas and abscesses in their lungs at necropsy. There was a significant effect of bacterial dose on the number of bacterial isolated from the lungs and there was a significant (P<0.01) effect of treatment eith antibiotic +SLO on bacterial recoveries for the high dose group. This difference was not evident at the lower bacterial challenge dose, perhaps because of the variation in the antibiotic + SLO group (Group A). The pneumonia score for Group A was significantly lower than those of the other treatment groups (p=0.012). There was also a significant difference (p=0.001) in total collagen in the histology sections for Group A (antibiotic + SLO) compared to antibiotic therapy alone (Group C). While other pathological measures were not significantly different between the treatment groups, overall Group A (antibiotic therapy + SLO) tended to be lower.

*Discussion:* The overall impression based on the results of this experiment was that addition of SLO to the antibiotic regimen provided some added benefit, though this was not always statistically significant. However, if one looks at the pattern of the responses, the SLO + antibiotic therapy group (Group A) consistently exhibited better clinical responses compared to the other groups, including the antibiotic therapy alone group (Group C). Of particular interest were the lower bacterial counts (p=0.082) seen in Group A. This overall reduction in CFU/g suggests that combination treatment with SLO may have enhanced the efficacy of the antibiotic treatment by increasing penetration into the congested lung or individual granulomas. There was also less collagen deposited in the lungs of the foals in Group A (antibiotic + SLO), which could be associated with less scarring in the affected airway. The gross pathological findings also supported an enhancement effect of the combined antibiotic and SLO treatment as the combined treatment group (Group A) had significantly lower pneumonia scores. Here again, while other immunohistologic measures were not significantly different between the treatment groups, there was an overall trend for Group A (antibiotic + SLO) to be lower in many cases. It is possible that the methods may not have been of sufficient sensitivity to identify a treatment effect as in some cases there was no difference between those groups receiving antibiotic and those not being treated with antibiotic. This may have been due to insufficient time being allowed for lesion resolution prior to necropsy.

The goal of this project was to determine if treatment of infected foals with SLO would enhance their response to standard antibiotic therapy. All foals exhibited multiple microgranulomas in their lungs at necropsy and showed gross signs of pneumonia. *R. equi* was isolated from the lungs of the infected foals. A dose-dependent relationship was observed between the number of bacteria cultured from the lung and the dose of *R. equi* administered. Antibiotic treatment significantly reduced the bacterial load in the lung and lessened clinical signs, as expected.

Together, these results indicate that a successful challenge model of acute Rhodococcal pneumonia in the foals was employed. However, it should be noted that the lesions were not typical of field cases which present as a chronic abscessating bronchopneumonia. While abcessation was noted in the infected foals, this was not the predominant lesion. For example, had the bacterial dose been lower and the incubation period longer, more typical lesions would have been expected, as noted elsewhere (Jacks, S., Giguere, S., 2010, Effects of inoculum size on cell-mediated and humoral immune responses of foals experimentally infected with Rhodococcus equi: A pilot study. Veterinary Immunology and Immunopathology 282-286).

No difference in effect with SLO combination therapy compared to antibiotic therapy alone was observed in the immune response to the bacterial infection. For example, no difference between treatment groups was observed in terms of the percentage of PBMC producing IFN-γ in response to the *R. equi* stimulation. This result indicates that the improved clinical responses were likely not due to an enhanced immune response to the bacterium, though the *in vitro* measure employed might not necessarily reflect the relevant immune effector mechanism *in vivo.*

*Conclusion:* The foregoing Example demonstrates that foals challenged with *R. equi* treated with a combination therapy comprising a standard antibiotic regimen (i.e., rifampin and clarithromycin) and SLO exhibited lowed bacterial counts in the lung tissue examined at necropsy compared to the foals receiving either antibiotic therapy alone or SLO therapy alone. This result suggests that the SLO in the combination therapy enhanced the efficacy of the antibiotic treatment by increasing penetration into the congested lung or individual granulomas. Thus, the inclusion of SLO in a therapeutic regimen for the treatment of bacterial infections associated with abscesses is specifically contemplated.

Numerous modifications and variations in the practice of the invention are expected to occur to those skilled in the art upon consideration of the presently preferred embodiments thereof. Consequently, the only limitations which should be placed upon the scope of the invention are those which appear in the appended claims.

## Claims

1. A composition for use in the treatment of a disorder associated with sequestered bacteria in a mammalian subject, the disorder is an infection selected from the group consisting of mastitis, a pyoderma, tuberculosis and pneumonia, **characterised in that** said composition comprises streptolysin O (SLO) and an antibiotic in amounts effective to treat the disorder in a combination therapy.

2. The composition for use according to claim 1, wherein the sequestered bacteria is selected from the group consisting of *H. influenza, N. meningitidis, S. pneumoniae, M. tuberculosis, M. pneumonia, S. aureus* and *R. equi.*

3. The composition for use according to claim 1 or 2, wherein the disorder is selected from the group consisting of tuberculosis and pneumonia.

4. The composition for use according to any one of claims 1-3, wherein the SLO is administered in a dosage amount ranging from 0.0032 to 50 units (2 units/0.05 ml) per day.

5. The composition for use according to any one of claims 1-3, wherein the SLO is administered in a dosage amount ranging from 0.01 to 10 units per day.

6. The composition for use according to any one of claims 1-5, wherein the antibiotic comprises an antibiotic selected from the group consisting of rifampin, ciproflaxin, enoxacin, gatifloxacin, levofloxacin, lomefloxacin, moxifloxacin, nalidixic acid, norflonacin, oflonacin, capreomycin, cycloserine, ethambutol, thionamide, isoniazid, pyrazinamide, streptomycin, rclarithromycin, ifapentine, chloramphenicol, metroidazole, thiamphenicol, ertapenem, doripenem, cilastatin, doxycycline, erythromycin, nafcillin, oxacillin, vancomycin, penicillin and combinations thereof.

7. The composition for use according to claim 2, wherein the sequestered bacteria is *R. equi.*

8. The composition for use according to claim 7, wherein the antibiotic comprises a combination of rifampin and clarithromycin.

9. A composition for use in the treatment of pneumonia in a mammalian subject, said composition comprising a combination therapy comprising streptolysin O (SLO) and an antibiotic therapy comprising rifampin and clarithromycin, wherein the combination therapy is administered in an amount effective to treat the pneumonia.

10. The composition for use according to claim 9, wherein the SLO is administered in a dosage amount ranging from 0.0032 to 50 units (2 units/0.05 ml) per day.

11. The composition for use according to claim 9, wherein the SLO is administered in a dosage amount ranging from 0.01 to 10 units per day.

12. The composition for use according to claim 1 comprising SLO in an amount from ranging from 0.0008 to 50 units.

13. The composition for use according to claim 1 comprising SLO in an amount ranging from 0.025 to 10 units.

## Patentansprüche

1. Zusammensetzung zur Verwendung bei der Behandlung einer Erkrankung im Zusammenhang mit sequestierten Bakterien bei einem Säuger, wobei die Erkrankung eine Infektion ist, ausgewählt aus der Gruppe, bestehend aus Mastitis, einer Pyodermie, Tuberkulose und Lungenentzündung, **dadurch gekennzeichnet, dass** die Zusammensetzung Streptolysin O (SLO) und ein Antibiotikum in Mengen umfasst, die wirksam sind, um die Erkrankung in einer Kombinationstherapie zu behandeln.

2. Zusammensetzung zur Verwendung nach Anspruch 1, wobei die sequestrierten Bakterien ausgewählt sind aus der Gruppe, bestehend aus *H. influenza, N. meningitidis, S. pneumoniae, M. tuberculosis, M. pneumoniae, S. aureus* und *R. equi.*

3. Zusammensetzung zur Verwendung nach Anspruch 1 oder 2, wobei die Krankheit ausgewählt ist aus der Gruppe, bestehend aus Tuberkulose und Lungenentzündung.

4. Zusammensetzung zur Verwendung nach einem der Ansprüche 1-3, wobei die SLO in einer Dosierungsmenge im Bereich von 0,0032 bis 50 Einheiten (2 Einheiten/0,05 ml) pro Tag verabreicht wird.

5. Zusammensetzung zur Verwendung nach einem der Ansprüche 1-3, wobei die SLO in einer Dosierungsmenge im Bereich von 0,01 bis 10 Einheiten pro Tag verabreicht wird.

6. Zusammensetzung zur Verwendung nach einem der Ansprüche 1-5, wobei das Antibiotikum ein Antibiotikum umfasst, ausgewählt aus der Gruppe, bestehend aus Rifampin, Ciproflaxin, Enoxacin, Gatifloxacin, Levofloxacin, Lomefloxacin, Moxifloxacin, Nalidixinsäure, Norflonacin, Oflonacin, Capreomycin, Cycloserin, Ethambutol, Thionamid, Isoniazid, Pyrazinamid, Streptomycin, Rclarithromycin, Ifapentin, Chloramphenicol, Metroidazol, Thiamphenicol, Ertapenem, Doripenem, Cilastatin, Doxycyclin, Erythromycin, Nafcillin, Oxacillin, Vancomycin, Penicillin und Kombinationen davon.

7. Zusammensetzung zur Verwendung nach Anspruch 2, wobei die sequestrierten Bakterien *R. equi* sind.

8. Zusammensetzung zur Verwendung nach Anspruch 7, wobei das Antibiotikum eine Kombination aus Rifampin und Clarithromycin umfasst.

9. Zusammensetzung zur Verwendung bei der Behandlung von Lungenentzündung bei einem Säuger, wobei die Zusammensetzung eine Kombinationstherapie umfasst, die Streptolysin O (SLO) und eine Antibiotikum-Therapie umfasst, die Rifampin und Clarithromycin umfasst, wobei die Kombinationstherapie in einer Menge verabreicht wird, die zur Behandlung der Lungenentzündung wirksam ist.

10. Zusammensetzung zur Verwendung nach Anspruch 9, wobei die SLO in einer Dosierungsmenge im Bereich von 0,0032 bis 50 Einheiten (2 Einheiten/0,05 ml) pro Tag verabreicht wird.

11. Zusammensetzung zur Verwendung nach Anspruch 9, wobei die SLO in einer Dosierungsmenge im Bereich von 0,01 bis 10 Einheiten pro Tag verabreicht wird.

12. Zusammensetzung zur Verwendung nach Anspruch 1, umfassend SLO in einer Menge im Bereich von 0,0008 bis 50 Einheiten.

13. Zusammensetzung zur Verwendung nach Anspruch 1, umfassend SLO in einer Menge im Bereich von 0,025 bis 10 Einheiten.

## Revendications

1. Composition pour une utilisation dans le traitement d'un désordre associé à une bactérie séquestrée chez un sujet mammifère, ce désordre étant une infection sélectionnée dans le groupe comprenant la mastite, la pyodermie, la tuberculose et la pneumonie, **caractérisée en ce que** ladite composition comprend de la streptolysine O (SLO) et un antibiotique en quantités efficaces pour traiter le désordre dans une thérapie de combinaison.

2. Composition pour une utilisation selon la revendication 1, dans laquelle la bactérie séquestrée est choisie dans le groupe constitué par *H. influenza, N. meningitidis, S. pneumoniae, M. tuberculosis, M. pneumonia, S. aureus* et *R. equi.*

3. Composition pour une utilisation selon la revendication 1 ou 2, dans laquelle le désordre est choisi dans le groupe constitué par la tuberculose et la pneumonie.

4. Composition pour une utilisation selon l'une quelconque des revendications 1 à 3, dans laquelle le SLO est administrée en une dose allant de 0,0032 à 50 unités (2 unités/0,05 ml) par jour.

5. Composition pour une utilisation selon l'une quelconque des revendications 1 à 3, dans laquelle la SLO est administrée en une dose allant de 0,01 à 10 unités par jour.

6. Composition pour une utilisation selon l'une quelconque des revendications 1 à 5, dans laquelle l'antibiotique comprend un antibiotique choisi dans le groupe constitué par la rifampine, la ciproflaxine, l'énoxacine, la gatifloxacine, la lévofloxacine, la loméfloxacine, la moxifloxacine, l'acide nalidixique, la norflonacine, l'oflonacine, la capréomycine, la cyclosérine, l'éthambutol, le thionamide, l'isoniazide, la pyrazinamide, la streptomycine, la relarithromycine, l'ifapentine, le choramphénicol, le métroidazole, le thiamphénicol, l'ertapénem, le doripénem, la cilastatine, la doxycycline, l'érythromycine, la nafcillin, l'oxacilline, la vancomycine, la pénicilline et des compositions de ceux-ci.

7. Composition pour une utilisation selon la revendication 2, dans laquelle la bactérie séquestrée est *R. equi.*

8. Composition pour une utilisation selon la revendication 7, dans laquelle l'antibiotique comprend une combinaison de rifampine et de clarithromycine.

9. Composition pour une utilisation dans le traitement de la pneumonie chez un mammifère, ladite composition comprenant une thérapie de combinaison comprenant la streptolysine O (SLO) et une thérapie antibiotique comprenant de la rifampine et de la clarithromycine, où le traitement de combinaison est administré en une quantité efficace pour traiter la pneumonie.

10. Composition pour une utilisation selon la revendication 9, dans laquelle la SLO est administrée en une quantité posologique allant de 0,0032 à 50 unités (2 unités/0,05ml) par jour.

11. Composition pour une utilisation selon la revendication 9, dans laquelle la SLO est administrée en une dose allant de 0,01 à 10 unités par jour.

12. Composition pour une utilisation selon la revendication 1, comprenant la SLO en une quantité allant de 0,0008 à 50 unités.

13. Composition pour une utilisation selon la revendication 1, comprenant la SLO en une quantité allant de 0,025 à 10 unités.
